# EUROPEAN PATENT SPECIFICATION

(11) **EP 1 366 775 B1**
(45) Date of publication and mention of the grant of the patent: **27.07.2005**
(21) Application number: 03011827.7
(22) Date of filing: 26.05.2003
(51) Int. Cl.: A61M 1/00, A61B 19/00

(54) **Surgical cassette**
Chirurgische Kassette
Cassette surgicale

(30) Priority: 28.05.2002 US 156175
(43) Date of publication of application: 03.12.2003
(73) Proprietor: Alcon Inc., 6331 Hunenberg (CH)
(72) Inventor: Gordon, Raphael, San Dimas, California 91773 (US); Oliveira, Mel M., Huntington Beach, California 92649 (US)
(74) Representative: Hanna, Peter William Derek

(56) References cited:
- EP-A- 0 320 168
- EP-A- 0 776 670
- US-A- 5 531 698

## Description

### Background of the Invention

This invention relates generally to the field of surgical cassettes and more particularly to an identification system for surgical cassettes.

The use of cassettes with surgical instruments to help manage irrigation and aspiration flows into and out of a surgical site are well-known. See, for example, U.S. Patent Nos. 4,493,695, 4,627,833 (Cook), 4,395,258 (Wang, et al.), 4,713,051 (Steppe, et al.), 4,798,850 (DeMeo, et al.), 4,758,238, 4,790,816 (Sundblom, et al.), 5,267,956, 5,364,342 (Beuchat), 6,036,458 (Cole, et al.) and 6,059,544 (Jung, et al.),

The fluidic performance of the surgical instrument is substantially affected by the fluidic performance of the cassette. As a result, current surgical instrumentation and cassettes are designed to work as an integral system, with the fluidic performance of the cassette designed to optimize the fluidic performance of the entire surgical system. Recent advances made in surgical instrumentation now allow the surgeon to manually or automatically control the operating parameters of the surgical instrumentation to a very fine degree. Specialized cassettes have been developed to allow the surgeon to capitalize on the advance control afforded by modern surgical instrumentation. The operating parameters of the surgical instrumentation, however, must be adjusted depending upon the cassette being used. One system, disclosed in U.S. Patent No. 6,059,544 (Jung, et al.), has a cassette with a series of frangible tabs that can be used to allow the instrument to recognize the type of cassette being used. While such a system works very well, and has been commercially successful, an alternative method for identifying the cassette that is somewhat easier and less expensive to manufacture is desirable.

Accordingly, a need exists for a cassette identification system that does not require the use of frangible tabs.

### Brief Summary of the Invention

The present invention improves upon the prior art by providing a cassette, the cassette having a series of identifying tabs that vary from opaque to translucent. By varying the opaqueness of the tabs, the surgical system can readily identify the type of cassette being used.

Accordingly, one objective of the present invention is to provide a surgical cassette that can be readily identified by the surgical instrument in which the cassette is used.

Another objective of the present invention is to provide a surgical cassette having a series of tabs that vary in opaqueness in a selected pattern.

These and other advantages and objectives of the present invention will become apparent from the detailed description and claims that follow.

### Brief Description of the Drawing

FIG. 1 is a schematic illustration of the system of the present invention.
FIG. 2 is a perspective view of a cassette suitable for use with the present invention.
FIG. 3 is a perspective view of a surgical console that may be used with the system of the present invention.

### Detailed Description of the Invention

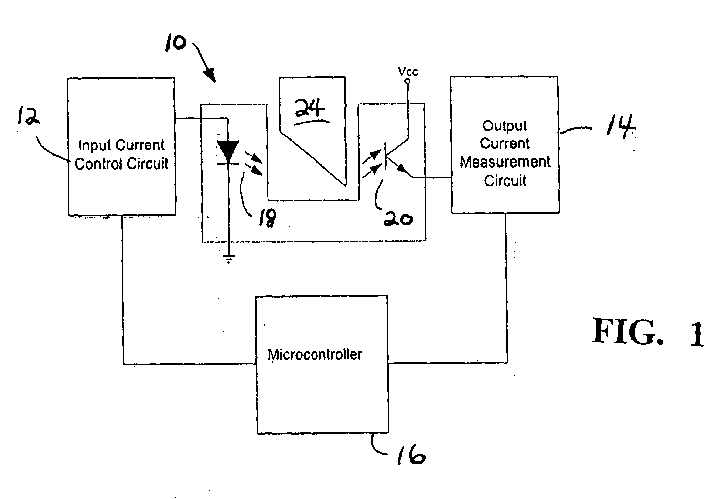

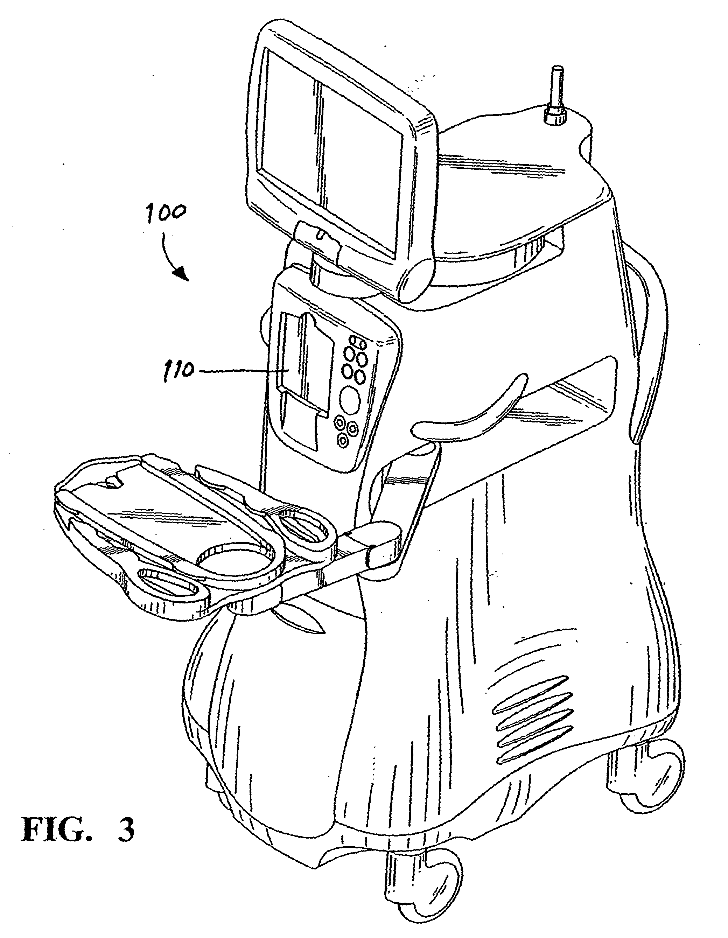

As best seen in FIG. 1, control system 10 generally includes input current control circuit 12 and output current measurement circuit 14, both of which being connected to microcontroller 16. Input current control circuit 12 controls the input current to light emitting diodes (LEDs) 18 and output current measurement circuit 14 measures the output current from photodetectors 20. Input current control circuit 12, output current measurement circuit 14 and microcontroller 16 may be any suitable hardware and/or software system, such systems being well-known in the art. The operation of LEDs 18 and photodetectors 20 being more fully explained in U.S. Patent No. 6,059,544 (Jung, et al.). System 10 may be included as part of the mechanical and electrical systems in any suitable surgical console, such as the console illustrated in FIG. 3.

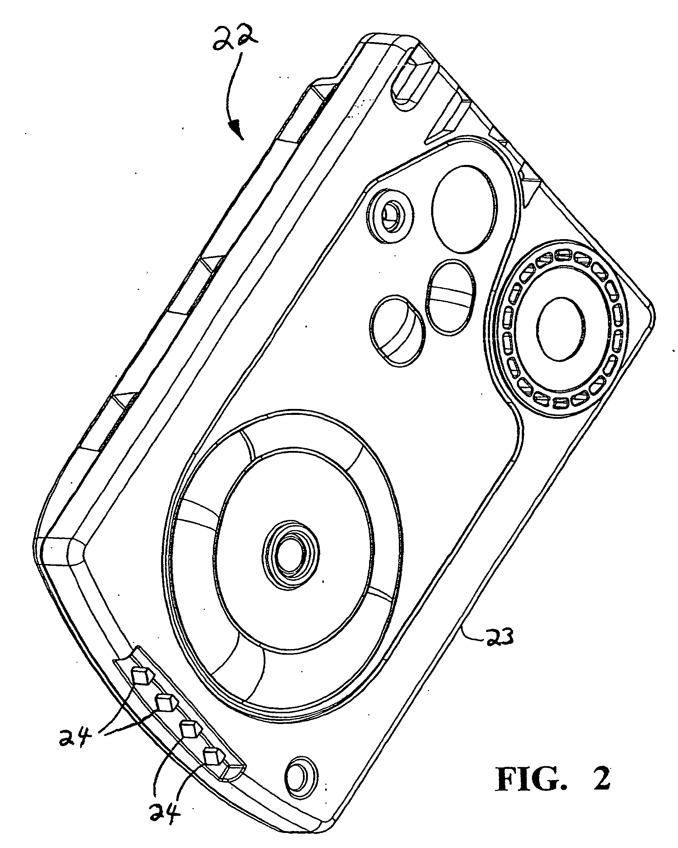

As best seen in FIG. 2, cassette 22 that may be used with system 10 of the present invention generally contains a plurality of tabs 24 projecting from housing 23. Tabs 24 may be generally of the shape described in U.S. Patent No. 6,059,544 (Jung, et al.) but may vary in opaqueness, from completely opaque to partially translucent to relatively clear. The opaqueness of tabs 24 may be used by system 10 in the manner described below. By varying the transmissibility of light through tabs 24, the number of possible distinct cassettes 22 may be increased without increasing the number of tabs 24.

In use, system 20 is calibrated by adjusting the output of LEDs 18, through input current control circuit 12, until a set output level in photodetectors 20 is achieved, as measured by output current measurement circuit 14. Upon insertion of cassette 22 into cassette receiving portion 110 of console 100, tabs 24 at least partially block the transmission of light from LEDs 18 to photodetectors 20, as shown in FIG. 1, and subsequently decrease the output of photodetectors 20. This drop in output from photodetectors 20 is sensed by output current measurement circuit 14, which relays this drop in output to microcontroller 16. Microcontroller 16 reads this decrease in the output of photodetectors 20 as an indication that cassette 22 is present in cassette receiving portion 110 of console 100. Microcontroller 16 may then instruct input current control circuit 12 to increase the output of LEDs 18 to the maximum level. Output current measurement circuit 14 then measures the output of photodetectors 20 with LEDs 18 at this maximum output level, and the output of photodetectors 20 is indirectly proportional to the opaqueness of tabs 24. In other words, the amount of light from LEDs 18 reaching photodetectors 20, and thus the output of photodetectors 20, will decrease proportionately with an increase in opaqueness of tabs 24. In this manner, the relative opaqueness of tabs 24 can be measured based on the variable amount of light reaching photodetectors 20 from LEDs 18. By varying the opaqueness of tabs 24 on cassette 22, the type of cassette 22 can be determined based on the opaqueness of tabs 24 and the pattern of variably opaque tabs 24 on cassette 22. For purposes of the present invention, each LED 18 is paired with a corresponding photodetector 20, and system 10 is arranged so that individual tabs 24 correspond to a LED/photodetector pair in a manner similar to that disclosed in U.S. Patent No. 6,059,544 (Jung, et al.).

This description is given for purposes of illustration and explanation. It will be apparent to those skilled in the relevant art that changes and modifications may be made to the invention described above without departing from its scope.

## Claims

1. A surgical cassette (22) adapted to be received by a surgical system, the cassette comprising a housing (23) and a plurality of identification tabs (24) projecting from the housing to be arranged in the path of a light source array when received by the system,
**characterized in that** the tabs (24) vary in opaqueness from completely opaque to partially translucent to relatively clear, such that the transmissibility of light through the tabs may be used to determine the type of cassette (22) based on the opaqueness of the tabs in a predetermined manner, wherein the number of distinctly identifiable cassettes (22) may be increased without increasing the number of identification tabs (24).

2. The surgical cassette of claim 1, wherein the tabs (24) are arranged in a selected pattern based on the opaqueness of the individual tabs.

## Patentansprüche

1. Chirurgische Kassette (22), die zur Aufnahme in einem chirurgischen System eingerichtet ist, wobei die Kassette ein Gehäuse (23) und mehrere Identifikationszungen (24) aufweist, die vom Gehäuse vorragen, so daß sie im Weg eines Lichtquellenfeldes angeordnet sind, wenn sie im System aufgenommen sind, **dadurch gekennzeichnet, daß** die Zungen (24) in ihrer Lichtundurchlässigkeit zwischen vollständig undurchlässig, teilweise durchlässig und relativ klar variieren, so daß die Lichtdurchlässigkeit durch die Zungen zur Bestimmung der Art der Kassette (22) basierend auf der Lichtundurchlässigkeit der Zungen in einer vorbestimmten Weise verwendet werden kann, wobei die Zahl von eindeutig identifizierbaren Kassetten (22) ohne eine Erhöhung der Zahl der Identifikationszungen (24) erhöht werden kann.

2. Chirurgische Kassette nach Anspruch 1, wobei die Zungen (24) in einem ausgewählten Muster basierend auf der Lichtundurchlässigkeit der einzelnen Zungen angeordnet sind.

## Revendications

1. Cassette chirurgicale (22) adaptée pour être reçue par un système chirurgical, la cassette comprenant un boîtier (23) et un ensemble de pions d'identification (24) faisant saillis à partir du boîtier, à agencer sur le trajet d'un réseau de source de lumière lorsqu'elle est reçue par le système,
**caractérisée en ce que** les pions (24) varient en opacité, à partir de complètement opaque à partiellement translucide jusqu'à relativement transparent, de sorte que la transmission de lumière à travers les pions peut être utilisée, afin de déterminer le type de cassette (22), en se basant sur l'opacité des pions d'une manière prédéterminée, dans laquelle le nombre de cassettes identifiables de manière distincte (22) peut être augmenté, sans accroître le nombre de pions d'identification (24).

2. Cassette chirurgicale selon la revendication 1, dans laquelle les pions (24) sont agencées selon un motif sélectionné, basé sur l'opacité des pions individuels.
